# EUROPEAN PATENT SPECIFICATION

(11) **EP 3 466 941 B1**
(45) Date of publication and mention of the grant of the patent: **05.08.2020**
(21) Application number: 17802678.7
(22) Date of filing: 18.05.2017
(51) Int. Cl.: C07D 319/06

(54) **PROCESS FOR PRODUCING CYCLIC ACETAL COMPOUND**
VERFAHREN ZUR HERSTELLUNG EINER CYCLISCHEN ACETALVERBINDUNG
PROCÉDÉ POUR PRÉPARER UN COMPOSÉ ACÉTAL CYCLIQUE

(30) Priority: 26.05.2016 JP 2016105538
(43) Date of publication of application: 10.04.2019
(73) Proprietor: Mitsubishi Gas Chemical Company, Inc., Tokyo 100-8324 (JP)
(72) Inventor: SATO, Hideyuki, Niigata-shi Niigata 950-3112 (JP); OKAMOTO, Atsushi, Niigata-shi Niigata 950-3112 (JP)
(74) Representative: Müller-Boré & Partner Patentanwälte PartG mbB
(86) International application number: PCT/JP2017/018729
(87) International publication number: WO 2017/204086

(56) References cited:
- EP-A1- 1 775 291
- WO-A1-2014/104341
- WO-A1-2014/171511
- CA-A1- 2 019 929
- JP-A- H0 338 585
- JP-A- S59 134 788
- JP-A- S59 148 776
- JP-A- 2005 239 599
- JP-A- 2007 099 681
- JP-A- 2016 013 986

## Description

The present invention relates to a process for producing a cyclic acetal compound.

Acetalization has been used for a long time as a technique to protect a highly reactive hydroxyl group or amino group by a carbonyl group or to protect a carbonyl group by a hydroxyl group. Acetal compounds have been utilized as synthetic intermediates for pharmaceuticals or agricultural chemicals. Moreover, acetal compounds themselves have been widely used as organic solvents, synthetic lubricating oils, surface active agents, etc.

As general synthesis methods for acetal compounds, dehydration reaction from a carbonyl compound and an alcohol using an acid catalyst and transacetalation through an acetal compound composed of a lower alcohol using an acid catalyst are widely known. In either case, the reaction is an equilibrium reaction, and therefore, in order to increase a reaction conversion, an operation to remove water or a lower alcohol, each being formed as a byproduct during the reaction, becomes necessary. For removing water, a reactive distillation method in which an organic solvent that is water-insoluble or sparingly water-soluble and forms an azeotropic mixture together with water is selected as a reaction solvent for the acetalization and only water of the separated two phases in the decanter tank is removed from the reaction system is generally utilized. For removing a lower alcohol, a method in which a distillation column is installed in the reactor and only a lower alcohol formed is removed from the reaction system is a general method.

Industrially, for the purpose of enhancing chemical stability or facilitating handling in use, many carbonyl compounds and alcohols that are raw materials of acetal compounds take the form of water-containing products, that is, mixtures thereof with water. For example, formaldehyde is widely distributed as its aqueous solution (formalin) having a concentration of about 40%, and glutaraldehyde is widely distributed as its aqueous solution having a concentration of about 2 to 20%. In order to enhance stability, 3-hydroxy-2,2-dimethyl-propionaldehyde (also known as: hydroxypivalaldehyde) is produced as a water-containing product having a concentration of 30 to 80%. Moreover, grape sugar (D-glucose) containing about 9% of water of crystallization and an aqueous solution product of D-sorbitol having a concentration of 70% are used as a sweetener and a pharmaceutical raw material, respectively.
Patent Document 1: Japanese Patent Application Laid-Open No. H11-228566
Patent Document 2: National Publication of International Patent Application No. 2006-515576
Patent Document 3: Japanese Patent Application Laid-Open No. H03-38585

Non Patent Document 1: Ettore Santoro, Journal of the Chemical Society, Perkin Transactions 11, Vol. 3, pp. 189-192, 1978

Carbonyl compounds that are raw materials of acetal compounds have high reactivity, and even by heating in the absence of a catalyst or even in long-term storage at ordinary temperature, byproducts are sometimes formed. For example, it is known that from an aldehyde compound represented by the following general formula (1), the corresponding ester compound (compound represented by the following general formula (4)) is formed through dimerization called Tishchenko reaction. It is further known that from the aldehyde compound, an alcohol and a carboxylic acid are formed through disproportionation of two molecules called Cannizzaro reaction. Moreover, it is reported that when a carbonyl compound has a hydroxyl group in the same molecule, the compound is dimerized or made to have a higher molecular weight through a hemiacetal linkage like 3-hydroxy-2,2-disubstituted-propionaldehyde (See non patent document 1). In this case, if acetal synthesis is carried out using an aldehyde containing the resulting dimer or high-molecular weight form as a raw material, a progress of desired acetalization is sometimes inhibited.

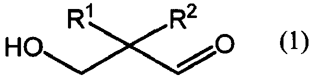

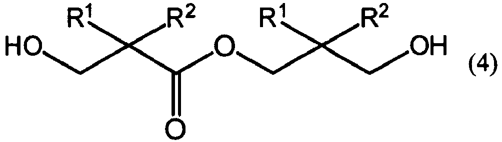

It is thought that in the case where an acetal compound is synthesized using, as a raw material, a mixture containing water together with an alcohol and/or a carbonyl compound (water-containing product), if water derived from the raw material can be removed in advance, there are advantages in the industrial production, such as shortening of the reaction time of the acetalization reaction and improvement in reactor efficiency. However, in the method in which an acetal raw material is dissolved in a water-insoluble or sparingly water-soluble organic solvent to perform two-phase separation, the method being an industrially possible dehydration method, another vessel different from the reaction vessel becomes necessary. In an air drying method, a reduced pressure method, a heating method, etc. that are general physical dehydration methods, it takes a long time to dehydrate. Moreover, such physical dehydration methods cannot be used in the case where water is added in order to enhance stability. Then, actually, a water-containing product that is a raw material of an acetal compound is introduced as it is into an acetal reaction apparatus and water derived from the raw material is removed before the acetalization reaction, or water formed by the acetalization reaction and water derived from a raw material are removed at the same time, in many cases (See patent documents 1 and 2).

However, when a highly reactive carbonyl compound is used as a raw material of an acetal compound, such a byproduct as described above is formed even in the dehydration step of removing water derived from the raw material. The byproduct remains as it is in the system of the acetalization reaction. Moreover, physical properties of the acetal compound and those of the byproduct come close to each other though it depends on the type of an alcohol selected as a raw material. As a result, it may become difficult to separate the acetal compound and the byproduct from each other by a distillation purification method or a recrystallization method, each being a general purification method. For example, in a patent document 3, synthesis of cyclic acetal from water-containing crude 2,2-dialkyl-3-hydroxypropanal and a 1,3-propandiol analogue is reported, but there is no description about a byproduct, and in all examples, distillation purification is carried out. As a result, in such a technique as described in the patent document 3, there occur problems such that the yield of a desired acetal compound decreases and complicated purification facilities are required.

The present invention has been made in the light of the above circumstances, and an object of the present invention is to provide a production process in which when an acetal compound is synthesized using a mixture containing a predetermined carbonyl compound that is a raw material together with water, formation of a byproduct is suppressed and a desired acetal compound is obtained in high purity by an industrially producible means.

The present inventors have earnestly studied, and as a result, they have found that in the case where a cyclic acetal compound is synthesized using a mixture containing an aldehyde compound that is a raw material and water in the presence of an acid catalyst, if acetalization reaction is carried out in the presence of water derived from the mixture, formation of a byproduct is suppressed and a desired acetal compound is obtained in high purity, and they have accomplished the present invention.

That is to say, the present invention is as follows.
[1] A process for producing a cyclic acetal compound represented by the following general formula (3), comprising a step of producing a cyclic acetal compound represented by the following general formula (3) by subjecting a compound A represented by the following general formula (1) and a compound B represented by the following general formula (2) to cyclodehydration reaction in a system comprising a mixture of the compound A and water, an acid catalyst and the compound (B), wherein a content of water contained in the mixture is 20% by mass or more based on the total amount of the water and the compound A, in the step of producing a cyclic acetal compound, water contained in the mixture and water formed by the cyclodehydration reaction are removed from the system at the same time: 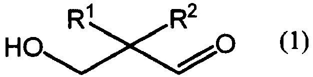 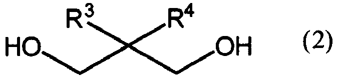 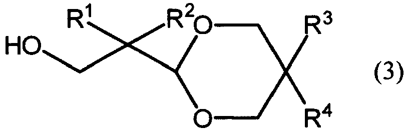 wherein R¹, R², R³ and R⁴ each independently represent a straight-chain or branched alkyl group having 1 to 6 carbon atoms or a hydroxymethyl group, and wherein in the step of producing a cyclic acetal compound, water contained in the mixture and water formed by the cyclodehydration reaction are removed from the system at the same time through azeotropy of an organic compound and water.
[2] The process for producing a cyclic acetal compound according to [1], wherein the organic compound is one or more selected from the group consisting of pentane, hexane, heptane, octane, benzene, toluene, xylene, cyclohexane, ligroin and petroleum ether.
[3] The process for producing a cyclic acetal compound according to any one of [1] to [2], wherein the compound A is 3-hydroxy-2,2-dimethyl-propionaldehyde.
[4] The process for producing a cyclic acetal compound according to any one of [1] to [3], wherein the compound B is one or more compounds selected from the group consisting of 2,2-dimethyl-1,3-propanediol, 2,2-diethyl-1,3-propanediol, 2-methyl-2-propyl-1,3-propanediol, 2-butyl-2-ethyl-1,3-propanediol, trimethylolethane, trimethylolpropane and pentaerythritol.
[5] The process for producing a cyclic acetal compound according to any one of [1] to [4], wherein the compound B is one or more compounds selected from the group consisting of 2,2-dimethyl-1,3-propanediol and 2-butyl-2-ethyl-1,3-propanediol.

According to the present invention, a production process in which when an acetal compound is synthesized using a mixture containing a predetermined carbonyl compound that is a raw material together with water, formation of a byproduct is suppressed and a desired acetal compound is obtained in high purity by an industrially producible means can be provided.

Hereinafter, an embodiment for carrying out the present invention (referred to as "the present embodiment" simply hereinafter) will be described in detail. The present embodiment below is an example to describe the present invention, and the present invention is not limited to the present embodiment only. The process for producing an acetal compound of the present embodiment is one comprising a step of producing a cyclic acetal compound represented by the following general formula (3) by subjecting a compound A represented by the following general formula (1) (also referred to as a "raw material aldehyde" hereinafter) and a compound B represented by the following general formula (2) (also referred to as a "raw material alcohol" hereinafter) to cyclodehydration reaction in a system comprising a mixture of the compound A and water, an acid catalyst and the compound (B), wherein a content of water contained in the mixture is 20% by mass or more based on the total amount of the water and the compound A, and in the step of producing a cyclic acetal compound, water contained in the mixture and water formed by the cyclodehydration reaction are removed from the system at the same time. In the formulas (1), (2) and (3), R¹, R², R³ and R⁴ each independently represent a straight-chain or branched alkyl group having 1 to 6 carbon atoms or a hydroxymethyl group. Examples of the straight-chain or branched alkyl groups having 1 to 6 carbon atoms include methyl group, ethyl group, propyl group (n-propyl group or isopropyl group), butyl group (n-butyl group, sec-butyl group, isobutyl group or tert-butyl group), pentyl group (n-pentyl group, isopentyl group, sec-pentyl group, 3-pentyl group, tert-pentyl group or neopentyl group), and hexyl group (1-methylpentyl group, n-hexyl group, isohexyl group, sec-hexyl group, tert-hexyl group or neohexyl group).

In the step of producing a cyclic acetal compound by cyclodehydration reaction (referred to as "cyclodehydration step" hereinafter), the compound A and the compound B are subjected to cyclodehydration reaction in a system comprising a mixture of the compound A and water, the mixture comprising 20% by mass or more of water based on the total amount of the compound A and water, an acid catalyst and the compound B. The compound A is 3-hydroxy-2,2-disubstituted-propionaldehyde, and specific examples thereof include monoalcohol aldehydes, such as 3-hydroxy-2,2-dimethyl-propionaldehyde, 3-hydroxy-2,2-diethyl-propionaldehyde, 3-hydroxy-2-methyl-2-ethyl-propionaldehyde, 3-hydroxy-2-methyl-2-propyl-propionaldehyde, 3-hydroxy-2-methyl-2-butyl-propionaldehyde, 3-hydroxy-2-methyl-2-pentyl-propionaldehyde, 3-hydroxy-2-ethyl-2-propyl-propionaldehyde, 3-hydroxy-2-ethyl-2-butyl-propionaldehyde, 3-hydroxy-2-ethyl-2-pentyl-propionaldehyde, 3-hydroxy-2-ethyl-2-hexyl-propionaldehyde, 3-hydroxy-2-dipropyl-propionaldehyde, 3-hydroxy-2-propyl-2-butyl-propionaldehyde, 3-hydroxy-2-propyl-2-pentyl-propionaldehyde, 3-hydroxy-2-propyl-2-hexyl-propionaldehyde, 3-hydroxy-2-dibutyl-propionaldehyde, 3-hydroxy-2-butyl-2-pentyl-propionaldehyde, 3-hydroxy-2-butyl-2-hexyl-propionaldehyde, 3-hydroxy-2-dipentyl-propionaldehyde, 3-hydroxy-2-pentyl-2-hexyl-propionaldehyde, 3-hydroxy-2-dihexyl-propionaldehyde, and 3-hydroxy-2-methyl-2-hexyl-propionaldehyde; dialcohol aldehydes, such as 2,2-bishydroxymethyl-acetaldehyde, 2,2-bishydroxymethyl-propionaldehyde, 2,2-bishydroxymethyl-butyraldehyde, 2,2-bishydroxymethyl-valeraldehyde, and 2,2-bishydroxymethyl-hexylaldehyde; and trialcohol aldehydes, such as 2,2,2-trishydroxymethyl-acetaldehyde. From the viewpoint that the effect of the present invention is exerted more effectively and more surely, the monoalcohol aldehyde of the above compounds is preferably 3-hydroxy-2,2-dimethyl-propionaldehyde, 3-hydroxy-2,2-diethyl-propionaldehyde, 3-hydroxy-2-methyl-2-ethyl-propionaldehyde, 3-hydroxy-2-methyl-2-propyl-propionaldehyde, 3-hydroxy-2-methyl-2-butyl-propionaldehyde, 3-hydroxy-2-ethyl-2-butyl-propionaldehyde, 3-hydroxy-2-propyl-2-pentyl-propionaldehyde, or 3-hydroxy-2-methyl-2-hexyl-propionaldehyde; and more preferably 3-hydroxy-2,2-dimethyl-propionaldehyde. From the same viewpoint, the dialcohol aldehyde is preferably 2,2-bishydroxymethyl-acetaldehyde, 2,2-bishydroxymethyl-propionaldehyde, 2,2-bishydroxymethyl-valeraldehyde or 2,2-bishydroxymethyl-hexylaldehyde. Of the monoalcohol aldehydes, the dialcohol aldehydes and the trialcohol aldehydes, the monoalcohol aldehydes are preferable from the same viewpoint. The substituents bonded to the 2-position carbon atom of the propionaldehyde skeleton correspond to R¹ and R² in the general formula (3) representing a structure of the cyclic acetal compound.

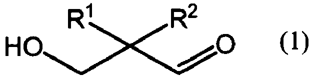

A process for producing 3-hydroxy-2,2-disubstituted-propionaldehyde that is the compound A is not particularly limited, and a compound produced by a hitherto known process can be used. For example, 3-hydroxy-2,2-dimethyl-propionaldehyde is known to be synthesized by aldol condensation from isobutyraldehyde and formalin. In this case, in order to enhance storage stability of the resulting 3-hydroxy-2,2-dimethyl-propionaldehyde, this compound is distributed in the market as a mixture containing 70 to 20% by mass of water based on the total amount of the compound and water. In the present embodiment, acetal synthesis can be carried out by utilizing, for example, such a mixture (water-containing product). The content of water in the mixture of the compound A and water is not particularly limited as long as it is 20% by mass or more, that is, 20% by mass or more but less than 100% by mass, based on the total amount of them, and for example, the content may be 20% by mass or more but 70% by mass or less, may be 20% by mass or more but 50% by mass or less, or may be 20% by mass or more but 40% by mass or less.

The compound B is 2,2-disubstituted-1,3-propandiol. Specific examples thereof include diols, such as 2,2-dimethyl-1,3-propanediol, 2,2-diethyl-1,3-propanediol, 2-methyl-2-ethyl-1,3-propanediol, 2-methyl-2-propyl-1,3-propanediol, 2-methyl-2-butyl-1,3-propanediol, 2-methyl-2-pentyl-1,3-propanediol, 2-ethyl-2-propyl-1,3-propanediol, 2-ethyl-2-butyl-1,3-propanediol, 2-ethyl-2-pentyl-1,3-propanediol, 2-ethyl-2-hexyl-1,3-propanediol, 2,2-dipropyl-1,3-propanediol, 2-propyl-2-butyl-1,3-propanediol, 2-propyl-2-pentyl-1,3-propanediol, 2-propyl-2-hexyl-1,3-propanediol, 2,2-dibutyl-1,3-propanediol, 2-butyl-2-pentyl-1,3-propanediol, 2-butyl-2-hexyl-1,3-propanediol, 2,2-dipentyl-1,3-propanediol, 2-pentyl-2-hexyl-1,3-propanediol, 2,2-dihexyl-1,3-propanediol, and 2-methyl-2-hexyl-1,3-propanediol; triols, such as trimethylolethane, trimethylolpropane, trimethylolbutane, trimethylolpentane and trimethylolhexane; and tetraols, such as pentaerythritol. From the viewpoint that the effect of the present invention is exerted more effectively and more surely, the diol of the above compounds is preferably 2,2-dimethyl-1,3-propanediol, 2,2-diethyl-1,3-propanediol, 2-methyl-2-ethyl-1,3-propanediol, 2-methyl-2-propyl-1,3-propanediol, 2-methyl-2-butyl-1,3-propanediol, 2-ethyl-2-butyl-1,3-propanediol, 2-propyl-2-pentyl-1,3-propanediol, or 2-methyl-2-hexyl-1,3-propanediol; and more preferably 2,2-dimethyl-1,3-propanediol or 2-ethyl-2-butyl-1,3-propanediol. From the same viewpoint, the triol is preferably trimethylolethane, trimethylolpropane, trimethylolbutane, trimethylolpentane or trimethylolhexane. Of the diols, the triols and the tetraols, the diols are preferable from the same viewpoint. The substituents bonded to the 2-position carbon atom of 1,3-propanediol correspond to R³ and R⁴ in the general formula (3) representing a structure of the cyclic acetal compound.

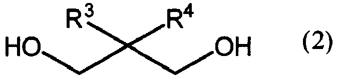

In the cyclodehydration step, the amount of the compound B used based on the amount of the compound A used is not particularly limited as long as it is an amount capable of producing a desired cyclic acetal compound. However, in the case where the product is subjected to the next reaction without being purified after the cyclodehydration reaction, an unreacted or excess compound B remains as it is in the product, and therefore, a bad influence is sometimes exerted on the next reaction. Also in the case where the product is purified after the cyclodehydration reaction and the unreacted portion is recovered, it is industrially advantageous that the unreacted portion is as small as possible. From these viewpoints, the amount of the compound B used based on the amount of the compound A used is preferably 1.00 equivalent or more but 3.00 equivalents or less on a molar basis, more preferably 1.05 equivalents or more but 2.00 equivalents or less on a molar basis.

The cyclic acetal compound represented by the following general formula (3) according to the present embodiment only needs to be one having the same R¹, R², R³ and R⁴ as R¹, R², R³ and R⁴ in the compound A and the compound B, and any combination is available.

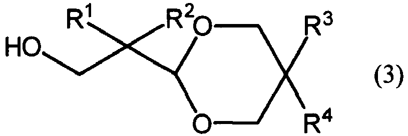

In the cyclodehydration step, the acid catalyst for use in the cyclodehydration reaction (acetalization reaction) may be a known acid catalyst and is not particularly limited. Specific examples of such acid catalysts include organic acids, such as paratoluenesulfonic acid and methanesulfonic acid, mineral acids, such as hydrochloric acid and sulfuric acid, and solid acid catalysts, such as Nafion (trade name, manufactured by Sigma-Aldrich Corporation) and a cation exchange resin. Of these, organic acids are more preferable, and paratoluenesulfonic acid is still more preferable, from the viewpoint that the effect of the present invention is exerted more effectively and more surely. The amount of the acid catalyst used in the cyclodehydration step is not particularly limited, but is preferably 0.00001 equivalent or more but 0.01 equivalent or less on a molar basis, based on the amount of the raw material aldehyde. From the viewpoint of the reaction time, the amount of the acid catalyst used is preferably 0.00001 equivalent or more, more preferably 0.0001 equivalent or more, on a molar basis, based on the amount of the raw material aldehyde. From the viewpoints of suppression of byproduct formation and removal of catalyst, the amount of the acid catalyst used is preferably 0.01 equivalent or less, more preferably 0.001 equivalent or less, on a molar basis, based on the amount of the raw material aldehyde.

In the present embodiment, an organic compound (except one corresponding to the organic compound of the aforesaid compounds) may be contained in the system. The organic compound employable herein is preferably an organic solvent that is liquid at room temperature and homogeneously dissolves the raw material aldehyde, the raw material alcohol and the resulting cyclic acetal compound at the temperature at the time of the cyclodehydration reaction, and is more preferably one capable of forming an azeotropic mixture together with water. From the viewpoint that the organic compound is desired to be water-insoluble or sparingly water-soluble and inert to the raw material aldehyde, the raw material alcohol and the resulting cyclic acetal compound, a hydrocarbon-based solvent is more preferably used as the organic compound. Examples of the hydrocarbon-based solvents include paraffins, aromatic hydrocarbons and alicyclic hydrocarbons. Specific examples of the hydrocarbon-based solvents include pentane, hexane, heptane, octane, benzene, toluene, xylene, cyclohexane, ligroin and petroleum ether. Such organic compounds (preferably hydrocarbon-based solvents) may be used singly, or may be used as a mixture of two or more thereof. Here, the expression "water-insoluble or sparingly water-soluble" refers to properties that the solubility in water at room temperature is less than 2 g/L.

The amount of the organic compound used is not particularly limited, but is preferably 10 parts by mass or more but 1000 parts by mass or less, more preferably 20 parts by mass or more but 500 parts by mass or less, still more preferably 30 parts by mass or more but 300 parts by mass or less, based on the total amount 100 parts by mass of the raw material aldehyde and the raw material alcohol. Since the amount of the organic compound used is in the above numerical range, water can be removed from the system more effectively and more surely when the organic compound can form an azeotropic mixture together with water.

In the cyclodehydration step of the present embodiment, the reaction temperature of the cyclodehydration reaction may be 50°C or higher but 180°C or lower, and is preferably 70°C or higher but 150°C or lower, though it depends on a boiling point of the organic compound when the organic compound is contained. When the reaction temperature is 50°C or higher, the reaction time can be further shortened, and when the reaction temperature is 180°C or lower, an undesired side reaction attributable to coloring can be suppressed more effectively and more surely. The reaction pressure in the cyclodehydration reaction is not particularly limited as long as it is a pressure at which the cyclodehydration reaction proceeds at the above-mentioned reaction temperature, and the reaction pressure may also be normal pressure, or in some cases, it is also effective to carry out the reaction under reduced pressure. The atmosphere around the reaction system at the time of this reaction is not particularly limited, and the reaction may be carried out in any of, for example, an air atmosphere, a nitrogen atmosphere and a stream of nitrogen. The reaction time only needs to be appropriately adjusted by the catalytic amount or the reaction temperature, but is preferably 2 hours or longer but 48 hours or shorter.

In the cyclodehydration step of the present embodiment, water contained in the mixture and water formed by the cyclodehydration reaction are removed from the system at the same time. A method to remove water from the system is not particularly limited, and a method to remove water formed in the dehydration reaction from the reaction system may be a generally known method. For example, the method may be a method in which an organic compound that is water-insoluble or sparingly water-soluble and is capable of forming an azeotropic mixture together with water is incorporated in the reaction system, then the organic compound and water are distilled off as azeotropic fractions, and thereafter only water of the separated two phases is removed from the system. In the case where an organic compound capable of forming an azeotropic mixture together with water is used, the temperature to remove water is not particularly limited as long as it is a temperature at which water and the organic compound are azeotroped with each other.

The cyclic acetal compound obtained by the production process of the present embodiment is subjected to appropriate post-treatments, such as neutralization, filtration, washing and concentration, and then further subjected to a known purification method, whereby the cyclic acetal compound can be isolated. Specific examples of such isolation methods include distillation, crystallization, adsorption treatment, column chromatography, preparative HPLC (liquid chromatography) and preparative gas chromatography. According to the production process of the present embodiment, a cyclic acetal compound can be obtained in high purity, and therefore, by carrying out only the post-treatments after the reaction, the cyclic acetal compound can be used for the next reaction or for the intended application without further carrying out an isolation operation of the cyclic acetal compound. According to the present embodiment, when a cyclic acetal compound is synthesized using a mixture of the raw material aldehyde and water, formation of a byproduct is suppressed and an acetal compound of high purity can be obtained.

### Examples

Hereinafter, the present invention will be more specifically described with reference to the examples, but the present invention is not particularly limited by the following examples. Unless otherwise noted, the representation "%" in the examples is on a mass basis. The content of water in the raw material and the reaction results were derived from the results determined by an internal standard method using gas chromatography (GC) and using a TCD detector.

### (Example 1)

642.3 g of a mixture of water and 3-hydroxy-2,2-dimethyl-propionaldehyde (hydroxypivalaldehyde, compound (1)) (mixture: manufactured by MITSUBISHI GAS CHEMICAL COMPANY, INC., content of water: 31%), 456.6 g of 2,2-dimethyl-1,3-propanediol (neopentyl glycol, compound (2), reagent manufactured by Tokyo Chemical Industry Co., Ltd.), 1414 g of toluene (reagent manufactured by Wako Pure Chemical Industries, Ltd.) and 0.42 g of paratoluenesulfonic acid monohydrate (reagent manufactured by Wako Pure Chemical Industries, Ltd.) were put into a 5-liter round-bottom flask, and they were heated to 90°C to 125°C at normal pressure to perform cyclodehydration reaction. While azeotroping water (containing at least water contained in the mixture and water formed by the reaction) in the system with toluene at that temperature, the water was removed from the system to the outside thereof using a Dean-Stark trap, and the reaction was continued until distillation of water stopped. GC measurement of the reaction solution after the removal of water resulted in 98.7% of 2-(5,5-dimethyl-[1,3]dioxan-2-yl)-2-methyl-propan-1-ol (compound (3)), 0.1% of 3-hydroxy-2,2-dimethyl-propionaldehyde and 0.5% of 3-hydroxy-2,2-dimethylpropyl-3-hydroxy-2,2-dimethylpropionate (referred to as "ester glycol" hereinafter). The reaction solution was concentrated and cooled, thereby obtaining 650.9 g of primary crystals of 2-(5,5-dimethyl-[1,3]dioxan-2-yl)-2-methyl-propan-1-ol. The reaction scheme of Example 1 is shown below.

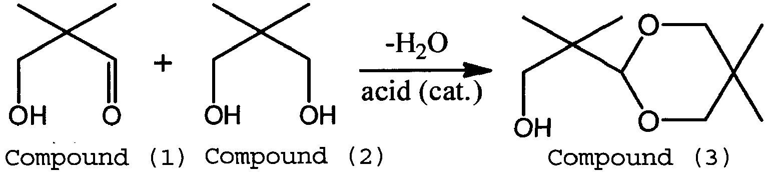

### (Comparative Example 1)

758.1 g of a mixture of water and 3-hydroxy-2,2-dimethyl-propionaldehyde (manufactured by MITSUBISHI GAS CHEMICAL COMPANY, INC., content of water: 31%) and 1000 g of toluene (reagent manufactured by Wako Pure Chemical Industries, Ltd.) were put into a 5-liter round-bottom flask, then they were heated to 90°C to 125°C at normal pressure, and while azeotroping water having been contained in the mixture with toluene, the water was removed from the system to the outside thereof using a Dean-Stark trap until distillation of water stopped. GC measurement of the liquid after the removal of water resulted in 82.1% of 3-hydroxy-2,2-dimethyl-propionaldehyde and 17.9% of ester glycol. After the interior of the flask was cooled down to 30°C, 221.5 g of 2,2-dimethyl-1,3-propanediol (reagent manufactured by Tokyo Chemical Industry Co., Ltd.) and 0.20 g of paratoluenesulfonic acid monohydrate (reagent manufactured by Wako Pure Chemical Industries, Ltd.) were newly added to the system, and they were heated to 100°C to 125°C again at normal pressure to perform reaction. While azeotroping water having been formed by the reaction with toluene at that temperature, the water was removed from the system to the outside thereof using a Dean-Stark trap, and the reaction was continued until distillation of water stopped. GC measurement of the reaction solution after the removal of water resulted in 88.8% of 2-(5,5-dimethyl-[1,3]dioxan-2-yl)-2-methyl-propan-1-ol, 0.2% of 3-hydroxy-2,2-dimethyl-propionaldehyde and 8.9% of ester glycol.

### (Comparative Example 2)

21.0 g of a mixture of water and 3-hydroxy-2,2-dimethyl-propionaldehyde (manufactured by MITSUBISHI GAS CHEMICAL COMPANY, INC., content of water: 31%), 15.2 g of 2,2-dimethyl-1,3-propanediol (reagent manufactured by Tokyo Chemical Industry Co., Ltd.) and 46.5 g of toluene (reagent manufactured by Wako Pure Chemical Industries, Ltd.) were put into a 0.2-liter round-bottom flask, then they were heated to 90°C to 125°C at normal pressure, and while azeotroping water having been contained in the mixture with toluene, the water was removed from the system to the outside thereof using a Dean-Stark trap until distillation of water stopped. GC measurement of the liquid after the removal of water resulted in 44.9% of 3-hydroxy-2,2-dimethyl-propionaldehyde, 52.1% of 2,2-dimethyl-1,3-propanediol and 3.1% of ester glycol.

### (Example 2)

Cyclodehydration reaction and removal of water from the system were carried out under the same conditions as in Example 1, except that 456.6 g of 2,2-dimethyl-1,3-propanediol was changed to 702.5 g of 2-ethyl-2-butyl-1,3-propanediol (reagent manufactured by Tokyo Chemical Industry Co., Ltd.). GC measurement of the reaction solution after the removal of water resulted in 94.8% (as a total value of isomers) of 2-(5-ethyl-5-butyl-[1,3]dioxan-2-yl)-2-methyl-propan-1-ol, 0.1% of 3-hydroxy-2,2-dimethyl-propionaldehyde and 0.5% of ester glycol. The reaction solution was subjected to alkali washing and then subjected to vacuum distillation, thereby obtaining 2-(5-ethyl-5-butyl-[1,3]dioxan-2-yl)-2-methyl-propan-1-ol (99.0% as a total value of isomers based on GC measurement). The reaction scheme of Example 2 is shown below.

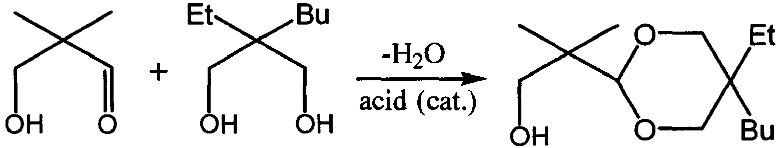

### (Comparative Example 3)

Reaction and removal of water from the system were carried out under the same conditions as in Comparative Example 1, except that 221.5 g of 2,2-dimethyl-1,3-propanediol was changed to 341.3 g of 2-ethyl-2-butyl-1,3-propanediol (reagent manufactured by Tokyo Chemical Industry Co., Ltd.). GC measurement of the liquid after the removal of water resulted in 87.0% (as a total value of isomers) of 2-(5-ethyl-5-butyl-[1,3]dioxan-2-yl)-2-methyl-propan-1-ol, 0.2% of 3-hydroxy-2,2-dimethyl-propionaldehyde and 7.8% of ester glycol.

### (Comparative Example 4)

Reaction and removal of water from the system were carried out under the same conditions as in Comparative Example 3, except that 15.2 g of 2,2-dimethyl-1,3-propanediol was changed to 23.1 g of 2-ethyl-2-butyl-1,3-propanediol (reagent manufactured by Tokyo Chemical Industry Co., Ltd.). GC measurement of the liquid after the removal of water resulted in 42.8% of 3-hydroxy-2,2-dimethyl-propionaldehyde, 53.2% of 2-ethyl-2-butyl-1,3-propanediol and 4.0% of ester glycol.

From the above results, it was understood that by utilizing the present invention in the synthesis of a cyclic acetal compound, formation of a byproduct was suppressed and a desired cyclic acetal compound was obtained in high purity.

The cyclic acetal compounds obtained by the production process of the present invention can be utilized as end-capping agents for polyester, polycarbonate, polyurethane, polyamide, polyoxymethylene and the like or monomer raw materials, and also as resin additives or intermediate raw materials for pharmaceuticals and agricultural chemicals. Since the cyclic acetal compounds obtained by the production process of the present invention have high purity, it is possible to subject the cyclic acetal compounds that remain unpurified to the reaction of the next step without providing a step of any particular purification such as distillation or recrystallization. The present invention has industrial applicability in such fields.

## Claims

1. A process for producing a cyclic acetal compound represented by the following general formula (3), comprising a step of producing a cyclic acetal compound represented by the following general formula (3) by subjecting a compound A represented by the following general formula (1) and a compound B represented by the following general formula (2) to cyclodehydration reaction in a system comprising a mixture of the compound A and water, an acid catalyst and the compound (B), wherein
a content of water contained in the mixture is 20% by mass or more based on the total amount of the water and the compound A,
in the step of producing a cyclic acetal compound, water contained in the mixture and water formed by the cyclodehydration reaction are removed from the system at the same time: 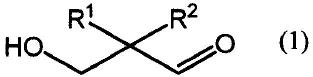 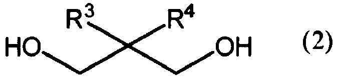 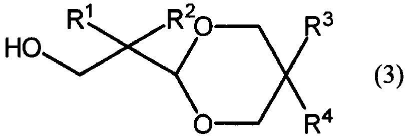 wherein R¹, R², R³ and R⁴ each independently represent a straight-chain or branched alkyl group having 1 to 6 carbon atoms or a hydroxymethyl group, and
wherein
in the step of producing a cyclic acetal compound, water contained in the mixture and water formed by the cyclodehydration reaction are removed from the system at the same time through azeotropy of an organic compound and water.

2. The process for producing a cyclic acetal compound according to claim 1, wherein the organic compound is one or more selected from the group consisting of pentane, hexane, heptane, octane, benzene, toluene, xylene, cyclohexane, ligroin and petroleum ether.

3. The process for producing a cyclic acetal compound according to any one of claims 1 and 2, wherein the compound A is 3-hydroxy-2,2-dimethyl-propionaldehyde.

4. The process for producing a cyclic acetal compound according to any one of claims 1 to 3, wherein the compound B is one or more compounds selected from the group consisting of 2,2-dimethyl-1,3-propanediol, 2,2-diethyl-1,3-propanediol, 2-methyl-2-propyl-1,3-propanediol, 2-butyl-2-ethyl-1,3-propanediol, trimethylolethane, trimethylolpropane and pentaerythritol.

5. The process for producing a cyclic acetal compound according to any one of claims 1 to 4, wherein the compound B is one or more compounds selected from the group consisting of 2,2-dimethyl-1,3-propanediol and 2-butyl-2-ethyl-1,3-propanediol.

## Patentansprüche

1. Verfahren zur Herstellung einer cyclischen Acetalverbindung, dargestellt durch die folgende allgemeine Formel (3),
umfassend einen Schritt des Herstellens einer cyclischen Acetalverbindung, dargestellt durch die folgende allgemeine Formel (3), durch Unterwerfen einer Verbindung A, dargestellt durch die folgende allgemeine Formel (1), und einer Verbindung B, dargestellt durch die folgende allgemeine Formel (2), einer Cyclodehydratisierungsreaktion in einem System, umfassend ein Gemisch der Verbindung A und Wasser, einen sauren Katalysator und die Verbindung (B),
wobei ein Gehalt an Wasser, enthalten in dem Gemisch, 20 Masse-% oder mehr, basierend auf der Gesamtmenge des Wassers und der Verbindung A, ist, in dem Schritt des Herstellens einer cyclischen Acetalverbindung Wasser, enthalten in dem Gemisch, und Wasser, gebildet durch die Cyclodehydratisierungsreaktion, aus dem System gleichzeitig entfernt werden: 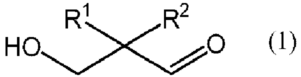 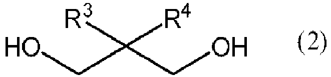 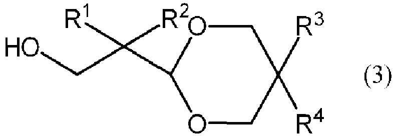
worin R¹, R², R³ und R⁴ jeweils unabhängig voneinander eine geradkettige oder verzweigtkettige Alkylgruppe mit 1 bis 6 Kohlenstoffatomen oder eine Hydroxymethylgruppe darstellen, und
wobei in dem Schritt des Herstellens einer cyclischen Acetalverbindung Wasser, enthalten in dem Gemisch, und Wasser, gebildet durch die Cyclodehydratisierungsreaktion, aus dem System gleichzeitig durch Azeotropie einer organischen Verbindung und Wasser entfernt werden.

2. Verfahren zur Herstellung einer cyclischen Acetalverbindung gemäß Anspruch 1, wobei die organische Verbindung eine oder mehrere, ausgewählt aus der Gruppe, bestehend aus Pentan, Hexan, Heptan, Octan, Benzol, Toluol, Xylol, Cyclohexan, Ligroin und Petrolether, ist.

3. Verfahren zur Herstellung einer cyclischen Acetalverbindung gemäß einem der Ansprüche 1 und 2, wobei die Verbindung A 3-Hydroxy-2,2-dimethyl-propionaldehyd ist.

4. Verfahren zur Herstellung einer cyclischen Acetalverbindung gemäß einem der Ansprüche 1 bis 3, wobei die Verbindung B eine oder mehrere Verbindungen, ausgewählt aus der Gruppe, bestehend aus 2,2-Dimethyl-1,3-propandiol, 2,2-diethyl-1,3-propandiol, 2-Methyl-2-propyl-1,3-propandiol, 2-Butyl-2-ethyl-1,3-propandiol, Trimethylolethan, Trimethylolpropan und Pentaerythritol, ist.

5. Verfahren zur Herstellung einer cyclischen Acetalverbindung gemäß einem der Ansprüche 1 bis 4, wobei die Verbindung B eine oder mehrere Verbindungen, ausgewählt aus der Gruppe, bestehend aus 2,2-Dimethyl-1,3-propandiol und 2-Butyl-2-ethyl-1,3-propandiol, ist.

## Revendications

1. Procédé de production d'un composé acétal cyclique représenté par la formule générale (3) suivante, comprenant une étape de production d'un composé acétal cyclique représenté par la formule générale (3) suivante en soumettant un composé A représenté par la formule générale (1) suivante et un composé B représenté par la formule générale (2) suivante à une réaction de cyclodéshydratation dans un système comprenant un mélange du composé A et d'eau, d'un catalyseur acide et du composé (B), dans lequel
la teneur en eau dans le mélange est de 20% en masse ou plus sur base de la quantité totale d'eau et du composé A,
dans l'étape de production d'un composé acétal cyclique, l'eau présente dans le mélange et l'eau formée par la réaction de cyclodéshydratation sont simultanément éliminées du système : 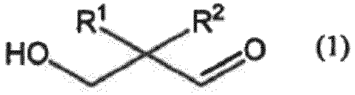 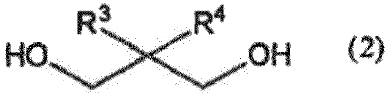 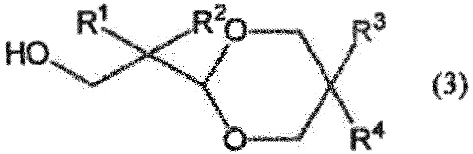
dans lequel R¹, R², R³ et R⁴ représentent chacun indépendamment, un groupe alkyle linéaire ou ramifié ayant 1 à 6 atomes de carbone ou un groupe hydroxyméthyle, et dans lequel
dans l'étape de production d'un composé acétal cyclique, , l'eau présente dans le mélange et l'eau formée par la réaction de cyclodéshydratation sont simultanément éliminées du système par azéotropie d'un composé organique et de l'eau.

2. Procédé de production d'un composé acétal cyclique selon la revendication 1, dans lequel le composé organique est un ou plusieurs choisis parmi le groupe consistant en le pentane, l'hexane, l'heptane, l'octane, le benzène, le toluène, le xylène, le cyclohexane, la ligroïne et l'éther de pétrole.

3. Procédé de production d'un composé acétal cyclique selon la revendication 1 ou 2, dans lequel le composé A est le 3-hydroxy-2,2-diméthylpropionaldéhyde.

4. Procédé de production d'un composé acétal cyclique selon l'une quelconque des revendications 1 à 3, dans lequel le composé B est l'un ou plusieurs composés choisis parmi le groupe consistant en du 2,2-diméthyl-1,3-propanediol, du 2,2-diéthyl-1,3-propanediol, du 2-méthyl-2-propyl-1,3-propanediol, du 2-butyl-2-éthyl-1,3-propanediol, du triméthyloléthane, du triméthylolpropane et du pentaérythritol.

5. Procédé de production d'un composé acétal cyclique selon l'une quelconque des revendications 1 à 4, dans lequel le composé B est l'un ou plusieurs composés choisis parmi le groupe consistant en du 2,2-diméthyl-1,3-propanediol et du 2-butyl-2-éthyl-1,3-propanediol.
